# EUROPEAN PATENT APPLICATION

(11) **EP 4 793 954 A1**
(43) Date of publication of application: **19.08.2026**
(21) Application number: 25158435.5
(22) Date of filing: 17.02.2025
(51) Int. Cl.: G16B 20/00, G16B 40/20, G16H 50/20, G16H 50/30

(54) **PROSTATE CANCER PREDICTION**

(71) Applicant: EDX Medical Ltd, Cambridge Cambridgeshire CB4 0WA (GB)
(72) Inventor: The designation of the inventor has not yet been filed
(74) Representative: Gill Jennings & Every LLP

(57) **Abstract**

A computer-implemented method of predicting the current and/or future status of a cancer in a subject suspected of having said cancer is provided. The method comprises (a) accessing a plurality of molecular marker datasets, wherein each molecular marker dataset represents the presence of one or more molecular markers from a respective omics class as determined within one or more biological samples of the subject, wherein the omics classes of the plurality of molecular marker datasets are different; (b) inputting each molecular marker dataset into a respective omics model to predict, for each omics class, a current and/or future status of the cancer in the subject, wherein each omics model is a trained machine learning or statistical model; and (c) providing an output prediction of the current and/or future status of the cancer in the subject, based on the predicted status of the cancer for each omics class obtained in step (b).

## Description

### BACKGROUND

According to the World Health Organisation, prostate cancer is the 4^{th} most common tumour type found in men and is the 8^{th} highest source of cancer-associated mortalities worldwide. Primarily affecting individuals between the ages of 45 and 85, the incidence of prostate cancer worldwide increases and varies with age, varies across geographical regions and may occur at different ages and with different frequency in various ethnicities. As such, prostate cancer presents itself as a heterogenous disease with variations in presentation across different demographics.

As individuals age, the prostate naturally enlarges and harmless, non-cancerous (benign) lesions can form. The majority of these usually do not develop further and therefore should be left under surveillance rather than being subject to 'premature' therapeutic interventions where possible, if identified by medical testing (Schlomm T, Weischenfeldt J, Korbel J, Sauter G. The aging prostate is never "normal": implications from the genomic characterization of multifocal prostate cancers. Eur Urol. 2015 Sep;68(3):348-50. doi: 10.1016/j.eururo.2015.04.012). Additionally, however, certain cells originating in the prostate can become cancerous (rather than being benign lesions), first growing into localised tumours with the possibility of further invasion of other tissues such as the bladder or rectum and metastasising via the bloodstream or lymphatic system into bones and other organs, ultimately causing death. While the exact cause of cancers originating in the prostate is unknown, risk factors related to such 'prostate cancer' include ethnicity, age, obesity, lifestyle and other genetic/epigenetic and environmental factors.

The most common form of prostate cancer is adenocarcinoma, which originates in the glandular cells responsible for secreting prostatic fluid. Specific genetic changes which may result in the development of prostate cancer include androgen receptor dysregulation, wherein mutations in the androgen receptor cause cell hypersensitivity to androgens or androgen-independent growth. Whilst initial prostate cancer growth is usually androgen hormone-driven and associated with elevated levels of prostate-specific antigen (PSA), this is not always the case and is highly variable with individuals and at the time or circumstances of testing. Elevated PSA levels may indicate the presence of prostate cancer but this may also result from benign conditions like an enlarged or inflamed prostate.

Once the 'cancerous' cells or lesions are identified, further confirmatory testing and treatment is initiated, both of which may lead to further changes in the characteristic biological processes driving further proliferation, leading to 'resistance' to the applied therapy, demanding ongoing biological monitoring and therapy changes in order to control the emergent cancer. Despite emerging knowledge there is currently no consensus on a validated clinical care pathway to reflect this evolving disease.

The conventional pathway for a prostate cancer diagnosis is as follows:
First, a preliminary clinical screening assessment takes place. This may involve assessing clinical symptoms and patient data (such as age and ethnicity) based on the experience drawn from aggregated information from diverse large population studies. The 'clinical assessment' is usually quickly followed by a single biochemical marker test being carried out on the subject. This is commonly the PSA test at a first instance, with this hierarchical combination providing baseline information on which the decision to refer a patient for disease confirmation is made. This further confirmation usually takes place through a physical examination such as MRI scan, or prostate biopsy. Decisions for these screening processes are often made with reference to aggregated cumulative data, which can introduce bias, and therefore incremental improvements become harder. The current methodology in these practices attempts to identify cancer as early as possible, with a focus on not missing anything and consequently being a broad and catch-all approach. The inclusion of all data in an incremental stepwise clinical decisioning pathway to predict cancer status and prognosis does not automatically lead to enhanced accuracy of the detection of presence or nature of any prostate cancer.

Following a positive diagnosis of prostate cancer, treatment selection is made, which may either comprise active and tailored treatment, or the patient being monitored under active surveillance. Optionally, further molecular characterisation may be made by taking additional tumour, blood, saliva or urine samples. These are subjected to biochemical analysis to further characterise the tumour. These further studies are often validated on data and algorithms produced from smaller studies for individual classes of markers and may also be drawn from more defined, pre-qualified patient studies, which may lead to overlooking biological complexity and the datasets not capturing the full variability of the disease across different populations.

The polygenic nature of prostate 'cancer' and the need to exclude the benign lesions means that typical single biomarker tests used in the diagnosis of prostate cancer are not always reliable. While many prostate cancers may be associated with elevated levels of prostate specific antigen (PSA) at an early stage or throughout, the growth of some prostate cancers are independent of the androgen pathways and thus are associated with low levels of PSA. These include a number of especially aggressive androgen-independent tumour variants, also known as castration-resistant prostate cancer (CRPC). PSA levels also vary between individuals and are influenced by non-cancer-related status. As such, the concentration of PSA protein alone is an unreliable predictor of the presence, or absence, of active prostate cancer.

The biology of prostate-originated cancer is variable and often changes in response to treatment, demanding ongoing biological monitoring and changing therapies to overcome resistance. Aggressive variants with neuro-endocrine differentiation are seen in an increasing number of patients appearing to respond to hormonal withdrawal therapy in the short term but then rapidly forming extensive visceral metastases leading to poor survival. This aggressive variant is not associated with elevated PSA and rapidly loses the typical cellular features of prostate cancer, evading detection by PSA testing and traditional histology.

Due to this, the diagnosis of prostate cancer is a complex task and each of a variety of independent investigations or diagnostic assays are employed sequentially over a period of time (months or years) to confirm or exclude the presence of active cancer. Undertaking a risk assessment at initial detection and diagnosis is crucial in the management of the disease. From this, various medical decisions can be made, such as the likelihood of disease progression and suitable treatments to manage and help eliminate the cancer.

At first instance, the outlook in prostate cancer diagnosis seems promising, with the 10-year survival proportion reaching 90% in recent years. One factoring contribution is the increase in incidence rates over the same period due to an increased volume of testing being undertaken. While mortality rates have seen a decline, such trends largely reflect incidence and survival trends, and this fall in mortality is likely a result of both rising incidence and rising survival (Office for National Statistics, 2017).

Therefore, the rise in diagnoses does not necessarily mean more individuals are developing clinically significant prostate cancer and can instead be explained by overdiagnosis rather than a true increase in disease burden. The need for a clear, clinically relevant definition of 'cancer' in / of the prostate is critical to enable comparison and integration of clinical evidence, excluding benign lesions as well as detecting those true cancers whose growth is independent of PSA and androgen-driven pathways. Such a definition is crucial for several reasons, including facilitating evidence comparison, distinguishing true cancers, excluding benign lesions, improving treatment strategies, enhancing research focus, and standardizing clinical trials

For example, the advent of the PSA test caused a huge increase in men undergoing screenings for prostate cancer. This caused an increase in the identification of 'early-stage' prostate tumours and therefore resulted in a 50% decrease in prostate cancer-associated deaths in the US ("Screening for prostate cancer: Are PSA blood tests reliable?" University of Chicago Medicine, 15 June 2021). However, PSA is limited in that it cannot reliably differentiate between non-risk benign or slow-growing cancers, and cancers which may potentially be deadly if left untreated. Additionally, around 70-80% of men with elevated PSA levels do not have cancer, whereas 20% of men with normal PSA levels do have prostate cancer ("Is PSA reliable?" Harvard Health, 26 February 2020). One outcome of this is over-diagnosis, which in addition to being unnecessarily consuming for healthcare resources, can lead to unnecessary invasive biopsies which risk causing impotence, incontinence, or bowel dysfunction.

The PSA test is usually the first stage in the current patient pathway for a prostate cancer diagnosis. After this, an individual may be subjected to a digital rectal examination (DRE), which is often recommended in combination with a PSA test for the detection of prostate cancer. However, recent analysis has shown that the diagnostic value offered by DRE was akin to the PSA test, and nor was any additional benefit shown by combining the two diagnostic tests (Matsukawa A et al. Comparing the Performance of Digital Rectal Examination and Prostate-specific Antigen as a Screening Test for Prostate Cancer: A Systematic Review and Meta-analysis. Eur Urol Oncol. 2024 Aug;7(4):697-704. doi: 10.1016/j.euo.2023.12.005).

Further tools have been developed which have been used to assess potentially dangerous prostate tumours. MRI and CT scans offer ways in which a tissue may be visualised in detail without being invasive. MRIs can offer between 74% and 94% sensitivity for prostate scans and therefore offer a reliable way that cancer may be detected and can help determine if a biopsy is needed ("How accurate is magnetic resonance imaging (MRI) in diagnosing prostate cancer: An in-depth analysis" Urologo Cumming, 3 September 2024). However, the changes observed over time by repeat MRI scans may not be accurate enough to indicate cancer progression and therefore analysis by these techniques can have limitations. They can provide good visualisation of localised prostate enlargement but do not provide biological or predictive information on the nature or likely progression of the disease.

Biopsies comprise the surgical sampling and histopathological examination of portions of the prostate to help detect cancer cells within the samples. Tissue biopsy of the prostate is the only clinically accepted method for definitive diagnosis of prostate cancer. These techniques, however, have limitations, with some 12-core biopsy approaches resulting in a false-negative frequency of 30% (Serefoglu EC, Altinova S, Ugras NS, Akincioglu E, Asil E, Balbay MD. How reliable is 12-core prostate biopsy procedure in the detection of prostate cancer? Can Urol Assoc J. 2013 May-Jun;7(5-6):E293-8. doi: 10.5489/cuaj.11224).

One problem with the current approach of prostate cancer diagnosis is that the initial focus is too broad, often with a positive detection being made for tumours which may not pose an immediate or future risk to a subject. Additionally, the multiple tests offered are all made in in isolation with different "definitions" of cancer result in a lack of certainty of diagnosis. Another important element of testing that is largely missed is the accurate determination of the absence of disease, not just the presence, at the time of testing. It is key that any test is qualified with a risk profile based on modifiable factors and inherited factors, which can help establish an appropriate surveillance strategy for individuals, rather than immediately subjecting them to further investigation treatment that is not warranted in the case of clear negative cancer biology. Finally, while many biomarkers are associated with the presence or severity of the disease, few have a direct causal role or are sufficiently predictive alone, to guide and inform the selection of treatments for an individual patient's specific prostate cancer.

Due to the increase in incidence of prostate cancer and the continued development of screening technologies, a heightened demand for prostate cancer diagnostics has been created, with a 12% compound annual growth rate projected for the period from 2024 to 2030 ("Global Prostate Cancer Diagnostics Market to Observe Stunning Growth at a CAGR of ~12% by 2030" DelveInsight Business Research, 25 September 2024).

Effective population-level management of prostate cancer would involve early detection and differentiation between benign tumours and aggressive cancers originating in the prostate by a reliable, non-invasive means, coupled with rapid guidance to select tailored treatment on an informed basis. Selection of optimal treatment would include but not be limited to tumour biology, age, family history and genetic risks , and other risk factors present. The regular monitoring of patients on therapy is also proposed as being key for detecting the emergence of treatment-induced resistance, as this secondary source are often fatal cancers that are commonly originating in the prostate. Additionally, there is a need for avoiding unnecessary investigations and invasive procedures, which wastes resources and can incur both financial and physical costs for individuals.

As such, there is a continued need for the development of new diagnostic pathways to aid the early and accurate diagnosis of prostate cancer in individuals to achieve desired improved medical outcomes across populations. In particular, there is a key clinical need for a truly predictive biomarker panel for prostate cancer treatment outcomes, rather than the current prognostic biomarkers which only offer a snapshot of current disease status. For example, while MRI and biopsy histology can identify cancerous growths (thereby providing a confirmation of the presence of the cancer), they each fail to provide the biological details required to select targeted treatment and are not sufficiently accurate to monitor changes in tumours over a clinically relevant time period in which an effective therapy might be deployed.

### SUMMARY OF INVENTION

In general terms, the present invention is directed to a "multi-omics" approach to the prediction of the current and/or future status of cancer, preferably prostate cancer, in a subject. The invention provides a method of identifying and/or monitoring a cancer or potential cancer in a subject. By using multiple distinct datasets obtained from a plurality of different "omics" domains (genomics, transcriptomics, epigenomics, and proteomics), we are significantly enhancing the potential for increased accuracy of prostate cancer detection and characterisation rather than relying on a single omics platform alone.

In accordance with a first aspect of the invention, there is provided a computer-implemented method of predicting the current and/or future status of a cancer in a subject suspected of having said cancer, comprising:
(a) accessing a plurality of molecular marker datasets, wherein each molecular marker dataset represents the presence of one or more molecular markers from a respective omics class as determined within one or more biological samples of the subject, wherein
   the omics classes of the plurality of molecular marker datasets are different;
(b) inputting each molecular marker dataset into a respective omics model to predict, for each omics class, a current and/or future status of the cancer in the subject, wherein each omics model is a trained machine learning or statistical model; and
(c) providing an output prediction of the current or future status of the cancer in the subject, based on the predicted status of the cancer for each omics class obtained in step (b).

**In** this way, by inputting a plurality of molecular marker datasets from different omics classes into a respective plurality of (e.g. distinct) omics models to generate respective current and/or future cancer status predictions for each omics class, the method of the present invention provides a multi-omics approach to cancer prediction in a subject, thereby providing the advantages outlined above. In other words, molecular marker data from each distinct omics class is analysed separately to provide a plurality of omic-specific predictions to thereby provide a more reliable prediction of the current or future status of the cancer. The present invention advantageously uses a machine learning approach to provide a prediction of the current or future state of the cancer, rather than state of the art methods that are prognostic and typically focussed on a single omics class.

The multi-omic approach of the present invention advantageously utilises the redundancy of markers from different omics classes reflecting the same or coincident biological pathways to reinforce the predictive reliability. The use of different omics classes may additionally provide useful information on the trajectory of the future progression of the identified emergent cancer: for example, epigenetic, transcript and proteomic data may provide information on the time course of the cancer compared to a "snapshot" using a single omics class.

Advantageously, the plurality of molecular marker datasets may be obtained substantially concurrently (e.g. from the same biological samples), providing an enhanced clinical outlook at a particular moment in the cancer development in the subject.

The cancer may be a specific or non-specific cancer. Preferably, the cancer is prostate cancer. The multi-omics predictive approach of the present invention is particularly advantageous for prostate cancer due to its anomalous nature and the difficulty in prostate cancer detection and characterisation as has been discussed herein. However, the method may be applied to pan-cancers (for example glioblastoma, ovarian, pancreatic), and it is envisaged may be applied to non-cancer diseases.

Preferably, the output of each omics model comprises a quantification, numerical score or categorisation indicative of the predicted status of the cancer in the subject. Examples of such predictive outputs include one or more of: a prediction of the presence or absence of the cancer in the subject; a prediction of the presence or absence of a clinically detectable form of the cancer in the subject (e.g. in the case of a non-haematological, solid cancer); a prediction of the severity of the cancer; a prediction of a risk category, such as a D'Amico risk category, a Gleason Grade Group or a PI-RADS (Prostate Imaging-Reporting and Data System) score in the case of prostate cancer, or equivalent categories of other cancers; a predicted prognosis of the cancer.

In preferred embodiments, the output of each omics model comprises a predicted risk score indicative of one or more clinical risk indicators (e.g. clinical endpoints), preferably wherein the one or more clinical risk indicators include one or more of:
(i) the presence of cancer in the subject
(ii) the presence of clinically-significant cancer in the subject
(iii) a risk category, such as a D'Amico risk category, Gleason Grade Group or PI-RADS score in the case of prostate cancer (or equivalent categories of other cancers).

The presence of clinically significant cancer in the patient may be defined by a clinical diagnosis threshold (e.g. a Gleason Grade Group greater than a predefined value in the case of prostate cancer), or by a medical imaging risk score (e.g. an MRI PI-RADS score greater than a predetermined threshold).

Preferably, the omics class of each of the molecular maker datasets is one of the following:
(i) genomics
(ii) transcriptomics
(iii) epigenomics
(iv) proteomics.

A genomics dataset typically comprises data representative of gene mutations and genetic variations in the patient. A transcriptomics dataset typically comprises gene expression data. An epigenomics dataset typically comprises DNA methylation index data. A proteomics dataset typically comprises protein markers quantitation. Each molecular marker dataset typically comprises numerical data such as count data and/or quantitative index data.

In preferred embodiments, the plurality of molecular marker datasets comprises a genomics dataset, a transcriptomics dataset, an epigenomics dataset, and a proteomics dataset. In other words, in preferred embodiments, the step (a) of accessing a plurality of molecular marker datasets comprises accessing each of a genomics dataset, a transcriptomics dataset, an epigenomics dataset, and a proteomics dataset.

Preferably, at least two of the molecular marker datasets are from the same biological sample of the subject. In this way, the present invention provides improved cancer status prediction by providing a multi-omics approach based on a single (or minimal number of) biological sample provided by the subject.

The invention may include the initial steps of:
providing one or more biological samples from the subject; and
obtaining the plurality of molecular marker datasets from the one or more biological samples.

The obtaining of the plurality of molecular marker datasets from the one or more biological samples is performed *ex-vivo.* The biological samples may be obtained from the subject following an initial screening (e.g. PSA screening), as is done using conventional techniques.

The one or more biological samples include one or more of: a blood sample, a urine sample, a saliva sample, a tumor sample. Preferably, the subject is a human (e.g. human patient). However, in other examples the subject may be an animal (in other words the present invention may be used for veterinary use).

The one or more molecular marker datasets may be obtained using techniques known in the art as have been discussed herein.

Step (c) of the method provides an output prediction of the current or future status of the cancer in the patient, based on the predicted status of the cancer for each omics class obtained in step (b). The output prediction may, in some embodiments, be each of the individual status predictions obtained from each omics model (e.g. predicted risk scores indicative of one or more clinical risk indicators). This may be useful information for a medical practitioner or the subject in and of themselves. The output prediction could comprise an average (e.g. mean) of the individual status predictions from each omics model. However, preferably, the step (c) of providing an output prediction of the status of the cancer in the patient comprises assigning a weighting to each predicted status to obtain the output prediction. This is a particularly advantageous feature of the present "multi-omics" approach, in which the use of data from the different biological domains - rather than from a single omics class - are combined to provide a more accurate and robust prediction of the cancer status. For example, the data from each omics class can be integrated or excluded and further weighted to identify and characterise the cancer type and active status. This optimisation involves the selection or exclusion of specific data inputs to provide a more reliable diagnosis. As such, decisions can instead be made on selectively aggregated or excluded data which may be optimised to answer specific questions regarding a cancer diagnosis.

The weighting may be dependent on the clinical risk indicator. For example, the weightings of the omics models risk scores for the presence of cancer in the final output prediction may be different from the weightings of the omics models risk scores for a different clinical risk indicator. In this way, the present invention provides a flexible way of predicting the status of the cancer, allowing a user such as a medical practitioner to obtain the most relevant, specific, information for the patient's requirements.

The output prediction may additionally be based on subject clinical characteristic data and/or imaging data. In some embodiments the output prediction may be in the form of a report, for example for review by a medical practitioner.

Preferably, the step (c) of providing an output prediction of the status of the cancer in the subject comprises inputting the predicted status of the cancer for each omics class into an ensemble model, preferably wherein the ensemble model is a trained machine learning or statistical model. The ensemble model may be configured to apply a weighting to each omics class prediction as discussed above. Preferably, the ensemble model is a regression model.

The input into the respective omics models and/or the ensemble model may comprise one or more of:
(i) subject clinical characteristic data
(ii) imaging data, preferably MRI data or ultrasound data.

The subject clinical characteristic data may include one or more of: patient age, patient sex, patient weight, patient ethnicity, PSA level, previous biopsies, familial cancers, smoking history, comorbidities. Such clinical characteristic data advantageously provide additional improvements to the accuracy of the status prediction. In embodiments, the clinical characteristic data and/or imaging data may be used as input to both the omics models and the ensemble model. The molecular marker datasets may be standardised against the clinical characteristic data.

The imaging data (e.g. obtained from an MRI or ultrasound scan of the subject) may comprise an imaging risk score such as an MRI PI-RADS score. The imaging data may include data obtained from T1 weighted MRI and/or T2 weighted MRI, diffusion weighted imaging and/or dynamic contrast enhancement. Alternatively or additionally, the imaging data may comprise one or more of the following: (i) prostate / organ volume, (ii) density of non-cancerous lesions,(iii) presence and percentage of cancerous lesion - with biochemical data - comprising (iv) presence or absence and level of designated markers specifically associated with cancer growth and active cell division, (v) markers of proliferation and metastasis, (vi) presence or absence and level of designated markers specifically associated with aggressive cancer, in order to both characterise current disease status and predict future risk and course of disease progression. Preferably, said imaging data may be input into the ensemble model.

Preferably, each omics model is trained on a respective (e.g. distinct) reference dataset comprising a plurality of reference subject profiles including:
(i) reference molecular marker data indicative of the presence of one or more molecular markers of the respective omics class from one or more biological samples obtained from the reference subject; and
(ii) one or more labels indicative of the present or future status of the cancer in the reference subject.

Preferably, the one or more labels indicative of the status of the cancer in the reference subject comprise one or more clinical risk indicators such as one or more of: a D'Amico risk category, a Gleason Grade Group, a presence of cancer, an imaging risk score. The one or more labels may be obtained from biopsy outcomes and/or clinical risk categories. In embodiments, each reference subject profile may additionally comprise subject clinical characteristic data as described herein.

Preferably, each omics model comprises one of: a random forest model, a penalized regression model, a gradient boosted machine, a support vector machine or an artificial neural network. Typically, at least one (preferably each) omics model is a multivariate model (e.g. a multivariate random forest model) configured to predict different risk scores. However, in alternative embodiments, each molecular marker dataset may be input into a plurality of omics models trained for different clinical risk indicators.

In some embodiments, at least one (preferably each) of the plurality of molecular marker datasets is input into a respective prognostic model to generate one or more long term outcome predictions indicative of the future status of the cancer. The long term outcome predictions may be time-to-event predictions and may be combined with the predictions from each omics model to provide a combined risk prediction for each omics class. Typically the one or more prognostic models is a trained machine learning or statistical model, preferably an accelerated failure time (AFT) model. Preferred long term outcome predictions include estimated time to progression of the cancer, metastasis of the cancer, and estimated time to androgen resistance of the cancer.

In accordance with a second aspect of the invention there is provided a computer-implemented method of predicting the current and/or future status of a cancer in a subject suspected of having said cancer, comprising:
(a) accessing a multi-omics dataset comprising molecular marker data representing the presence of one or more molecular markers from a plurality of different omics class as determined within the biological sample obtained from the subject; and
(b) inputting the multi-omics dataset into a trained machine learning or statistical model to predict a current and/or future status of the cancer in the subject based on the plurality of different omics classes.

The trained machine learning or statistical model of the second aspect may comprise one of: a random forest model, a penalized regression model, a gradient boosted machine, a support vector machine or an artificial neural network.

Any of the aspect described above in relation to the first aspect of the invention may be applied to the second aspect of the invention.

In accordance with a third aspect of the invention there is provided a computer-implemented method of training a machine learning or statistical model to predict the current and/or future status of a cancer in a subject suspected of having said cancer, comprising:
(a) obtaining a reference dataset comprising a plurality of reference subject profiles including:
   (i) reference molecular marker data representative of the presence of one or more molecular markers from an omics class as determined within one or more biological samples obtained from the reference subject; and
   (ii) one or more labels indicative of the present or future status of the cancer in the reference subject; and
(b) applying a machine learning or statistical model to the reference dataset to learn a relationship between the reference molecular marker dataset and the one or more labels.

Further disclosed is a machine learning or statistical model trained using the method of the third aspect.

Preferably, according to any of the aspects of the invention, the plurality of molecular marker datasets comprises a genomics dataset comprising one or more of (preferably each of) the molecular markers listed in Table 1.

Preferably, according to any of the aspects of the invention, the plurality of molecular marker datasets comprises a transcriptomics dataset comprising one or more of (preferably each of) the molecular markers listed in Table 3.

Preferably, according to any of the aspects of the invention, the plurality of molecular marker datasets comprises an epigenomics dataset comprising one or more of (preferably each of) the molecular markers listed in Table 2.

Preferably, according to any of the aspects of the invention, the plurality of molecular marker datasets comprises a proteomics dataset comprising one or more of (preferably each of) the molecular markers listed in Table 4a.

In accordance with a further aspect of the invention there is provided a system for predicting the current and/or future status of a cancer in a subject, comprising at least one processor in communication with at least one memory device, the at least one memory device having stored thereon instructions for causing the at least one processor to perform the method according to any of the aspects of the invention described above.

Further disclosed herein is method of designing a treatment plan for a subject, comprising: performing the method of any of the aspect of the invention described above; and designing the treatment plan based on the predicted current or future status of the cancer (e.g. and its predicted development).

Further disclosed herein is a computer program product comprising instructions which, when executed by a computer, cause the computer to perform the method of any of the aspects of the invention described above.

Further disclosed herein is a computer-implemented method of predicting the current and/or future status of a cancer in a subject suspected of having said cancer, comprising:
(a) accessing a plurality of molecular marker datasets, wherein each molecular marker dataset represents the presence of one or more molecular markers from a respective omics class as determined within one or more biological samples of the subject, wherein
   the omics classes of the plurality of molecular marker datasets are different; and
(b) inputting each molecular marker dataset into a respective omics model to predict, for each omics class, a current or future status of the cancer in the subject, wherein each omics model is a trained machine learning or statistical model.

Such a method may be used in combination with any of the features discussed herein.

### BRIEF DESCRIPTION OF DRAWINGS

Embodiments of the invention will now be described with reference to the accompanying drawings, in which:-
Figure 1 is a flow diagram illustrating the principal steps of a method according to an embodiment of the invention;
Figure 2 schematically illustrates a flow diagram outlining a method for predicting the current or future status of prostate cancer in a subject according to an embodiment of the invention;
Figure 3Aschematically illustrates a flow diagram outlining a method for predicting the current or future status of prostate cancer in a subject according to a further embodiment of the invention;
Figure 3B schematically illustrates a machine learning pipeline for processing a molecular marker dataset from a single omics class, according to an embodiment of the invention;
Figure 4 schematically illustrates an example of a system suitable for implementing embodiments of the invention; and
Figure 5 schematically illustrates an example of a suitable server for implementing embodiments of the invention.

### DETAILED DESCRIPTION

The present invention makes use of a multi-omics approach to produce a risk score for the presence of prostate cancer. Examples of omics which may be used include genomics, epigenomics, transcriptomics, and proteomics.

### Genomics

Genomics is concerned with the identification of genes and how these influence the state of an individual, including genetic variants or mutations which may be associated with disease or certain genetic profiles which may respond to treatment in a specific way. These genetic aberrations may be present in genes passed from parents (hereditary) or they may occur in cells due to damage to cellular DNA during life as a result of spontaneous damage or similar loss of functional controls over cell division. This is elucidated principally by genome-wide association studies (GWAS), whereby a set of genetic variants (representing different individuals) are analysed to see if any distinct genetic variant is associated with a trait. For example, the presence of genes associated with an increased risk of cancer establishes a risk or chance that an individual may develop cancer in the future. Since these factors are genetic, they remain with an individual for life, and therefore knowledge of these factors can help inform lifestyle and medical choices to reduce the chance of those genes resulting in the establishment of disease. Certain techniques used in GWAS include genotype arrays, next-generation sequencing for whole-genome sequencing, and exome sequencing.

One example of identifying genomic biomarkers for prostate cancer may be done as follows. Tissue samples may be collected from a patient, and their genetic material extracted. From this, DNA sequencing may be carried out through, for example, next-generation sequencing techniques, to assess the genetic makeup of the sample from a patient which may include a sample directly from a cancer tumour. Bioinformatic tools can then be used, comparing the sequencing data of the patient to genetic databases of both healthy and affected individuals to identify any potential biomarkers relevant to prostate cancer such as specific mutations found within certain genes of interest. From this, candidate biomarkers may be validated in larger targeted and independent patient cohorts to confirm a specific association with prostate cancer. Once the clinical relevance of a genomic biomarker has been confirmed, this may be used to aid the prognosis of future prostate cancer patients and aid in predicting the trajectory of the disease.

**Table 1: List of genes which may represent sources of heritable germline mutations and which may form part of a prostate cancer panel designed for the detection and prediction of prostate cancer.**

| **Gene** | **ID/Acc. No.** | **Discovery Platform** | **Tissue** |
|---|---|---|---|
| **ATM** | ENSG00000149311 | GWAS | Blood |
| **BRCA1** | ENSG00000012048 | GWAS | Blood |
| **CHEK2** | ENSG00000183765 | GWAS | Blood |
| **EPCAM** | ENSG00000119888 | GWAS | Blood |
| **HOXB13** | ENSG00000159184 | GWAS | Blood |
| **MLH1** | ENSG00000076242 | GWAS | Blood |
| **MSH2** | ENSG00000095002 | GWAS | Blood |
| **MSH6** | ENSG00000116062 | GWAS | Blood |
| **NBN** | ENSG00000104320 | GWAS | Blood |
| **PALB2** | ENSG00000083093 | GWAS | Blood |
| **PMS2** | ENSG00000122512 | GWAS | Blood |
| **RAD51D** | ENSG00000185379 | GWAS | Blood |
| **TP53** | ENSG00000141510 | GWAS | Blood |
| **BRCA2** | ENSG00000139618 | GWAS | Blood |
| **ATR** | ENSG00000175054 | GWAS | Blood |
| **BRIP1** | ENSG00000136492 | GWAS | Blood |
| **GEN1** | ENSG00000178295 | GWAS | Blood |
| **NBN** | ENSG00000104320 | GWAS | Blood |

The genes described in Table 1 have been previously reported in peer-reviewed public literature.

### Epigenomics

Epigenomics encompasses reversible modifications made to DNA or DNA-associated proteins and reflects whether specific genes and associated regions of chromosomes are being actively transcribed. The expression of genes determines the phenotypical differentiation of the genetic potential of such cells. The two most common epigenetic modifications are DNA methylation and histone acetylation, and these covalent modifications inform the activity of a gene's transcription without changing its underlying genetic code. The most apparent example of this is observed in the process of cell differentiation, whereby two cells comprising the same genetic code may present a completely different phenotype depending on which genes have been switched 'on' or 'off' through epigenetic tags. The epigenetic profile of an individual is based on both environmental and genetic factors, and genes controlling the process of DNA methylation are subject to normal heritable traits. Hypermethylation of promoter regions inhibiting the expression of tumour suppressor genes resulting in an increased risk of the development of cancerous cells were the first epigenetic markers observed in human cancer. Epigenetic signatures can be tissue-specific, and therefore data generated by epigenetic investigations can help identify the specific markers associated with disease, independent of genetic variation.

The identification of relevant epigenomic biomarkers relevant to prostate cancer starts with the isolation of tissue material from a cancer patient and healthy control individuals. If DNA methylation patterns are desired to be studied, bisulfite sequencing or similar methods can be carried out to yield information about the methylation state of a DNA segment, such as a tumour suppressor gene, at the nucleotide level. Bioinformatic tools may then analyse this epigenetic data to confirm the presence of a potential biomarker, which may then be validated against larger patient cohorts. Once the clinical relevance of an epigenomic biomarker has been confirmed, this may be used to aid the prognosis of newly presenting prostate cancer patients, aid in predicting the trajectory of the disease and predict the response of the disease to specific therapies.

**Table 2: List of genes which may form part of a prostate cancer panel designed to identify distinct DNA methylation signatures for the detection and prediction of prostate cancer.**

| **Gene** | **Gene ID/Acc. No.** | **Discovery platform** | **Tissue** |
|---|---|---|---|
| **GSTP1** | ENSG00000084207 | Bisulfite methylation | Urine |
| **SFRP2** | ENSG00000145423 | TaqMan Low Density Arrays | Urine |
| **IGFBP3** | ENSG00000146674 | Bisulfite methylation | Urine |
| **IGFBP7** | ENSG00000163453 | Bisulfite methylation | Urine |
| **APC** | ENSG00000134982 | Bisulfite methylation | Urine |
| **PTGS2** | ENSG00000073756 | Bisulfite methylation | Urine |
| **APC** | ENSG00000134982 | - | Biopsy Prostate |
| **RASSF1** | ENSG00000068028 | - | Biopsy Prostate |

All these genes have been reported in peer-reviewed public literature as being clinically validated and associated with prostate cancer. All genes (apart from GSTP1) were identified in a multicenter prospective study of 463 men following analysis of prostate biopsies. Several but not all are included in the NCCN (National Comprehensive Cancer Network) Early Detection for Prostate Cancer Guidelines.

### Transcriptomics

Transcriptomics studies all RNA transcripts produced by the genome of any given organism. In any system, there may be a quantitative assessment of the transcripts present, which is indicative of how frequently a particular gene is being expressed, or a qualitative assessment of the transcripts present, which help determine which transcripts present and in which form they are in (i.e. specific splice variants or those which have been subjected to RNA editing). Transcriptomic approaches may be carried out at the bulk level, whereby whole cell populations are studied based on their expression profiles, which provides general information on a large sample of cells. Alternatively, single-cell transcriptomics are possible, which may elucidate information on rare cell types and cellular diversity. In addition to transcripts originating from protein-coding regions, transcriptomics can also identify RNAs produced from non-coding regions (i.e. those that do not get translated into a protein). These non-coding RNA transcripts are involved in translation processes (i.e. rRNA and tRNA), gene regulation (i.e. miRNAs) and RNA splicing. Just with protein-coding RNA transcripts, the dysregulation of non-coding RNA transcripts has been implicated in disease, and hence the study of this subset of RNAs can further uncover relevant therapeutic targets and mechanisms.

Transcriptomic profiling starts with RNA extraction from confirmed cancer tissues and control tissues, followed by RNA sequencing to measure gene expression levels across the transcriptome in the respective tissues and cells. Analysis of the transcriptomic data usually involves investigating differential expression of particular genes of interest which could serve as transcriptomic biomarkers for prostate cancer. Upon the identification of a potential biomarker, this can be validated against independent cohorts to confirm its significance and potential use in diagnosis and treatment options. Once the clinical relevance of a transcriptomic biomarker has been confirmed, this may be used to aid the prognosis of future prostate cancer patients and aid in predicting the trajectory of the disease.

**Table 3: List of genes for which transcriptomic profiles may form part of a prostate cancer panel for the detection and prediction of prostate cancer.**

| **Gene** | **Gene ID/Acc. No.** | **Discovery Platform** | **Tissue** |
|---|---|---|---|
| **TMPRSS2-ERG** | ENSG00000184012, ENSG00000157554 | RNA-SEQ | Urine |
| **SCHLAP1** | ENSG00000281131 | RNA-SEQ | Urine |
| **OR51E2** | ENSG00000167332 | RNA-SEQ | Urine |
| **APOC1** | ENSG00000130208 | RNA-SEQ | Urine |
| **PCAT14** | ENSG00000280623 | RNA-SEQ | Urine |
| **CAM KK2** | ENSG00000110931 | RNA-SEQ | Urine |
| **PCA3 (LncRNA)** | ENSG00000225937 | RNA-SEQ | Urine |
| **NKAIN1** | ENSG00000084628 | RNA-SEQ | Urine |
| **B3GNT6** | ENSG00000198488 | RNA-SEQ | Urine |
| **TFF3** | ENSG00000160180 | RNA-SEQ | Urine |
| **SPON2** | ENSG00000159674 | RNA-SEQ | Urine |
| **PCGEM1** | ENSG00000227418 | RNA-SEQ | Urine |
| **TRGV9** | ENSG00000211695 | RNA-SEQ | Urine |
| **TMSB15A** | ENSG00000158164 | RNA-SEQ | Urine |
| **ERG** | ENSG00000157554 | RNA-SEQ | Urine |
| **KLK4** | ENSG00000167749 | RNA-SEQ | Urine |
| **HOXC6** | ENSG00000197757 | RNA-SEQ | Urine |
| **KLK3 (Reference=PSA)** | ENSG00000142515 | RNA-SEQ | Urine |
| **DLX1** | ENSG00000144355 | RNA-SEQ | Urine |
| **AZGP1** | ENSG00000160862 | RNA-SEQ | Prostate Biopsy |
| **KLK2** | ENSG00000167751 | RNA-SEQ | - |
| **SRD5A2** | ENSG00000277893 | RNA-SEQ | - |
| **FAM13C** | ENSG00000148541 | RNA-SEQ | - |
| **FLNC** | ENSG00000128591 | RNA-SEQ | - |
| **GSN** | ENSG00000148180 | RNA-SEQ | - |
| **TPM2** | ENSG00000198467 | RNA-SEQ | - |
| **GSTM2** | ENSG00000213366 | RNA-SEQ | - |
| **TPX2** | ENSG00000088325 | RNA-SEQ | - |
| **BGN** | ENSG00000182492 | RNA-SEQ | - |
| **COL1A1** | ENSG00000108821 | RNA-SEQ | - |
| **SFRP4** | ENSG00000106483 | RNA-SEQ | - |
| **ARF1** | ENSG00000143761 | RNA-SEQ | - |
| **ATP5E** | ENSG00000124172 | RNA-SEQ | - |

The genes TMPRSS2-ERG, SCHLAP1, OR51E2, APOC1, PCAT14, CAMKK2, PCA3 (LncRNA), NKAIN1, B3GNT6, TFF3, SPON2, PCGEM1, TRGV9, TMSB15A, ERG, KLK4, HOXC6, KLK3 (Reference=PSA) and DLX1 have been identified as important transcriptomic biomarkers from a discovery cohort comprising 220 normal (benign) prostate tissue subjects, 71 low-grade cancer subjects, 484 high-grade cancer subjects; 761 high-grade cancer subjects (JAMA Oncol. 2024;10(6):726-736. doi:10.1001/jamaoncol.2024.0455, 18 April 2024).

The genes AZGP1, KLK2, SRD5A2, FAM13C, FLNC, GSN, TPM2, GSTM2, TPX2, BGN, COL1A1, SFRP4, ARF1 and ATP5E were identified as important transcriptomic biomarkers in a cohort sample of 501 patients (127 patients who experienced clinical recurrence and 374 patients who did not have a recurrence) who underwent radical prostatectomy between 1987 and 2004, and controls were selected at random from the 2,514 patients who did not experience clinical recurrence (Michael A. Brooks et al., GPS Assay Association With Long-Term Cancer Outcomes: Twenty-Year Risk of Distant Metastasis and Prostate Cancer-Specific Mortality. JCO Precis Oncol 5, 442-449(2021) doi: 10.1200/PO.20.00325).

### Proteomics

Proteomics is characterised by the large-scale study of the complete set of proteins in a biological system. The function, interaction, composition and structure of small peptides to large macromolecular protein complexes can be measured to illustrate how protein composition differs from cell to cell and has great importance in understanding disease at a molecular level. Proteins are the end-products or key outputs of most biochemical pathways, and their presence is one of the most reliable indicators of cell biology, including confirming the specifics of disease. By the time proteins are expressed in detectable amounts, the disease has essentially been "realised". Therefore, highly accurate and sensitive methods for studying proteins are essential. Proteomics has been vastly aided by the development of accurate and high-throughput instruments and technologies including mass spectrometry techniques, which has allowed the determination of protein mass and identity, including further analysis of post-translational modifications made to proteins which modulate their function *in vivo.*

After extracting relevant samples, the characterisation of proteins in select tissues and cells may be caried out through protein arrays, mass spectrometry, or gel electrophoresis techniques. Analysis of proteins may be quantitative by confirming their presence or absence with respect to a control sample, or quantitative in measuring their concentration or level compared to a control sample. Once a potential biomarker is found, this can be validated with comparison to larger independent cohorts, and from this their clinical relevance can be measured. Once the clinical relevance of a proteomic biomarker has been confirmed, this may be used to aid the prognosis of future prostate cancer patients and aid in predicting the trajectory of the disease.

**Table 4a: List of genes whose resultant proteins may form part of a prostate cancer panel for the detection and prediction of prostate cancer.**

| **PCa Outcome** | **Gene** | **UniProt ID** | **Tissue** | **Drug target** |
|---|---|---|---|---|
| **Early onset** | **MSMB** | P08118 | blood | No |
| | **PLG** | P00747 | blood | No |
| | **EHBP1** | Q8NDI1 | blood | No |
| | **TPM3** | P06753 | blood | Phenethyl Isothiocyanate |
| | **PYY** | P10082 | blood | - |
| | **POGLUT3** | Q7Z4H8 | blood | No |
| | **SERPINA3** | P01011 | blood | Zinc, Acetate, Chloride, Sulphate |
| | **PPA2** | Q9H2U2 | blood | No |
| **Aggressive** | **MSMB** | P08118 | blood | No |
| | **TNFRSF6B** | 095407 | blood | No |
| | **PPA2** | Q9H2U2 | blood | No |
| | **C4A** | P0C0L4 | blood | FDA approved drug targets, Systemic lupus erythematosus |
| | **TPM3** | P06753 | blood | Phenethyl Isothiocyanate |
| | **PRSS3** | P35030 | blood | 4-(1,3,2-DIOXAXABOROLAN-2-YLOXY).... |
| | **C2** | P06681 | blood | No |
| **Overall** | **MMP7** | P09237 | blood | No |
| | **TNFRSF10B** | 014763 | blood | No |
| | **SERPINA1** | P01009 | blood | No |
| | **NT5C2** | P49902 | blood | No |
| | **CREBL1/ATF6B** | Q99941 | blood | No |
| | **CREB3L4** | Q8TEY5 | blood | No |
| | **IFNA14** | P01570 | blood | No |
| | **ISLR2** | Q6UXK2 | blood | No |
| | **FAP** | Q12884 | blood | No |
| | **CDH17** | Q12864 | blood | No |
| | **DNER** | Q8NFT8 | blood | No |
| | **ICAM4** | Q14773 | blood | No |
| | **ADAMTS8** | Q9UP79 | blood | No |
| | **CXCL6** | P80162 | blood | No |
| | **TRAF2** | Q12933 | blood | No |
| | **GZMB** | P10144 | blood | No |
| | **F3** | P13726 | blood | FDA approved drug target |
| | **IL18RAP** | 095256 | blood | No |
| | **CRTAC1** | Q9NQ79 | blood | No |
| | **CD34** | P28906 | blood | No |
| | **IL20** | Q9NYY1 | blood | No |
| | **SPINK5** | Q9NQ38 | blood | No |
| | **SELP** | P16109 | blood | FDA approved drug target |
| | **HK2** | P52789 | blood | No |
| | **MIC1/RMC1** | Q96DM3 | blood | No |
| | **THBS1** | P07996 | blood | No |
| | **CTSD** | C9JH19 | blood | No |
| | **KLK3** | P07288 | blood/urine | FDA approved drug target |

Protein biomarkers, such as those found in Table 4a, have been reported and may be found through conventional techniques known in the art and new, emerging methods.

The genes listed in Table 4a for which the associated proteins are known as being associated with early onset and aggressive characteristics, in addition to MMP7, TNFRSF10B, SERPINA1, NT5C2, CREBL1/ATF6B, CREB3L4, IFNA14 and ISLR2 were identified from 20,907 cases and 289,710 controls from FinnGen and the UK Biobank, 1,872 African-ancestry participants obtained from the database of Genotypes and Phenotypes (dbGaP) (project#31553), and 10,363 cases with 10,986 controls from ELLIPSE Oncoarray using prostate cancer meta-analysis and genotyping (db#phs001120) (Desai T et al. Identifying proteomic risk factors for overall, aggressive, and early onset prostate cancer using Mendelian Randomisation and tumour spatial transcriptomics. EBioMedicine. 2024 Jul;105:105168. doi: 10.1016/j.ebiom.2024.105168).

FAP, CDH17, DNER, ICAM4, ADAMTS8, CXCL6, TRAF2, GZMB, F3, IL18RAP, CRTAC1, CD34, IL20, SPINK5, SELP, HK2, MIC1/RMC1 were identified in a cohort of 1477 cancer patients from 12 cancer types (U-CAN cohort, 28631533, EPN Uppsala 2010-198), and 74 healthy patients from the Wellness cohort (Swedish Sci-LifeLab SCAPIS Wellness Profiling (S3WP) (Álvez M et al. Next generation pan-cancer blood proteome profiling using proximity extension assay. Nat Commun. 2023 Jul 18;14(1):4308. doi: 10.1038/s41467-023-39765-y).

**Table 4b: List of genes provided in Table 4a with information on their putative biological pathway and functions in disease, source (https://v22.proteinatlas.org).**

| **Gene** | **Pathway/Disease/Function** |
|---|---|
| **MSMB** | Microseminoprotein beta, synthesized by the epithelial cells of the prostate gland and secreted into the seminal plasma. The expression of the encoded protein is found to be decreased in prostate cancer. Prostate cancer - lipid metabolism, upregulated in prostate cancer, lung cancer, colorectal cancer, breast cancer. |
| **PLG** | Plasminogen, blood coagulation, fibrinolysis, hemostasis, tissue remodeling, hydrolase, protease, serine protease, cancer-related genes, disease variant, FDA approved drug targets, thrombophilia. |
| **EHBP1** | EH domain-binding protein 1, endocytosis, protein transport. A single nucleotide polymorphism in this gene is associated with an aggressive form of prostate cancer. |
| **TPM3** | Tropomyosin alpha-3 chain, actin-binding, muscle protein, cancer-related genes, disease variant, nemaline myopathy, proto-oncogene. |
| **PYY** | Peptide YY, hormone, binding of the encoded peptides to these receptors mediates regulation of pancreatic secretion, gut mobility and energy homeostasis. |
| **POGLUT3** | Protein O-glucosyltransferase 3, protein glucosyltransferase that catalyses the transfer of glucose from UDP-glucose to a serine residue, prognostic marker in urothelial cancer (unfavourable) and liver cancer (unfavourable). |
| **SERPINA3** | Alpha-1-antichymotrypsin, prognostic marker in breast cancer (favourable), protease inhibitor, Serine protease inhibitor. |
| **PPA2** | Inorganic pyrophosphatase 2, Hydrolase, mitochondrial, prognostic marker in renal cancer (favourable), breast cancer (unfavourable) and colorectal cancer (favourable). |
| **MSMB** | Microseminoprotein beta, synthesized by the epithelial cells of the prostate gland and secreted into the seminal plasma. The expression of the encoded protein is found to be decreased in prostate cancer. Prostate cancer - lipid metabolism, upregulated in prostate cancer, lung cancer, colorectal cancer, breast cancer. |
| **TNFRSF6B** | TNF receptor superfamily member 6b, tumor necrosis factor receptor superfamily member 6B, receptor protein that is involved in apoptosis, upregulated in acute myeloid leukemia, myeloma, diffuse large B-cell lymphoma, chronic lymphocytic leukemia, ovarian cancer, lung cancer. |
| **PPA2** | Inorganic pyrophosphatase 2, hydrolase, mitochondrial, prognostic marker in renal cancer (favourable), breast cancer (unfavourable) and colorectal cancer (favourable). |
| **C4A** | Complement C4-A, blood group antigen, complement pathway, immunity, inflammatory response, innate immunity, disease variant, FDA approved drug targets, systemic lupus erythematosus. |
| **TPM3** | Tropomyosin 3, tropomyosin alpha-3 chain, actin-binding, muscle protein, prognostic marker in liver cancer (unfavourable), ovarian cancer (favourable), renal cancer (unfavourable), pancreatic cancer (unfavourable) and lung cancer (unfavourable). |
| **PRSS3** | Serine protease 3, trypsin-3, hydrolase, protease, serine protease, prognostic marker in endometrial cancer (unfavourable). |
| **C2** | Complement C2, hydrolase, protease, serine protease, complement pathway, immunity, innate immunity, prognostic marker in renal cancer (unfavourable), upregulated in diffuse large B-cell lymphoma, ovarian cancer. |
| **MMP7** | Matrix metallopeptidase 7, hydrolase, metalloprotease, protease, collagen degradation, prognostic marker in liver cancer (unfavourable) and lung cancer (unfavourable), upregulated in myeloma, ovarian cancer, chronic lymphocytic leukemia, lung cancer, endometrial cancer. |
| **TNFRSF10B** | TNF receptor superfamily member 10b, tumour necrosis factor receptor superfamily member 10B, apoptosis, prognostic marker in renal cancer (unfavourable), upregulated in acute myeloid leukemia, myeloma, chronic lymphocytic leukemia, diffuse large B-cell lymphoma, lung cancer. |
| **SERPINA1** | Serpin family A member 1, alpha-1-antitrypsin, protease inhibitor, serine protease inhibitor, acute phase, blood coagulation, haemostasis, prognostic marker in breast cancer (favourable) and colorectal cancer (favourable). |
| **NT5C2** | 5'-nucleotidase, cytosolic II, cytosolic purine 5'-nucleotidase, allosteric enzyme, hydrolase, transferase, nucleotide metabolism. |
| **CREBL1/ATF6B** | Activator, DNA-binding, transcription, transcription regulation, unfolded protein response. |
| **CREB3L4** | CAMP responsive element binding protein 3 like 4, transcription, transcription regulation, unfolded protein response, prognostic marker in endometrial cancer (favourable), group enriched (breast cancer, endometrial cancer, prostate cancer). |
| **IFNA14** | Interferon alpha 14, cytokine. |
| **ISLR2** | Immunoglobulin superfamily containing leucine rich repeat 2, Developmental protein, Required for axon extension during neural development, upregulated in Breast cancer, Prostate cancer |
| **FAP** | Fibroblast activation protein alpha, prolyl endopeptidase FAP, prognostic marker in head and neck cancer (unfavourable), upregulated in prostate cancer, breast cancer, endometrial cancer, chronic lymphocytic leukemia. |
| **CDH17** | Cadherin 17, cell adhesion, transport, calcium, metal-binding, upregulated in prostate cancer, breast cancer, endometrial cancer, colorectal cancer. |
| **DNER** | Delta/notch like EGF repeat containing, Delta and Notch-like epidermal growth factor-related receptor, activator, receptor, Notch signalling pathway, upregulated in prostate cancer, breast cancer, glioma, endometrial cancer, cervical cancer. |
| **ICAM4** | Intercellular adhesion molecule 4 (Landsteiner-Wiener blood group), blood group antigen, cell adhesion, upregulated in myeloma, breast cancer, prostate cancer, endometrial cancer. |
| **ADAMTS8** | ADAM metallopeptidase with thrombospondin type 1 motif 8, A disintegrin and metalloproteinase with thrombospondin motifs 8, heparin-binding, hydrolase, metalloprotease, protease, upregulated in prostate cancer, chronic lymphocytic leukemia, breast cancer, cervical cancer, endometrial cancer, disease prediction model prostate. |
| **CXCL6** | C-X-C motif chemokine ligand 6, antibiotic, antimicrobial, cytokine, heparin-binding, chemotaxis, upregulated in prostate cancer, disease prediction model prostate cancer |
| **TRAF2** | TNF receptor associated factor 2, transferase, apoptosis, UBL conjugation pathway, upregulated in acute myeloid leukemia, chronic lymphocytic leukemia, prostate cancer, myeloma, diffuse large B-cell lymphoma, glioma, breast cancer, disease prediction model prostate cancer. |
| **GZMB** | Granzyme B, hydrolase, protease, serine protease, apoptosis, cytolysis, upregulated in myeloma, acute myeloid leukemia, diffuse large B-cell lymphoma, chronic lymphocytic leukemia, prostate cancer, breast cancer, disease model prostate cancer. |
| **F3** | Coagulation factor III, tissue factor, blood coagulation, haemostasis, cancer-related genes, FDA approved drug targets, upregulated in chronic lymphocytic leukemia, breast cancer, prostate cancer, disease prediction model prostate cancer. |
| **IL18RAP** | Interleukin 18 receptor accessory protein, hydrolase, receptor, inflammatory response, upregulated in cervical cancer, prostate cancer, disease prediction model prostate cancer. |
| **CRTAC1** | Cartilage acidic protein 1, upregulated in prostate cancer, breast cancer, endometrial cancer, chronic lymphocytic leukemia, disease prediction model prostate cancer. |
| **CD34** | CD34 molecule, cell adhesion, upregulated in chronic lymphocytic leukemia, breast cancer, prostate cancer, endometrial cancer, disease prediction model prostate cancer. |
| **IL20** | Interleukin 20, pro-inflammatory and angiogenic cytokine, upregulated in prostate cancer, disease prediction model prostate cancer. |
| **SPINK5** | Serine peptidase inhibitor Kazal type 5, protease inhibitor, serine protease inhibitor, upregulated in prostate cancer, cervical cancer, breast cancer, endometrial cancer, disease prediction model prostate cancer. |
| **SELP** | Selectin P, cell adhesion, cancer-related genes, FDA approved drug targets, upregulated in lung cancer, colorectal cancer. |
| **HK2** | Hexokinase 2, allosteric enzyme, kinase, transferase, glycolysis, prognostic marker in renal cancer (unfavourable) and liver cancer (unfavourable). |
| **MIC1/RMC1** | Regulator of MON1-CCZ1, autophagy, prognostic marker in liver cancer (unfavourable). |
| **THBS1** | Thrombospondin 1, heparin-binding, cell adhesion, unfolded protein response, prognostic marker in renal cancer (unfavourable) and stomach cancer (unfavourable). |
| **CTSD** | Cathepsin D, aspartyl protease, hydrolase, protease, prognostic marker in renal cancer (favourable) and colorectal cancer (unfavourable). |
| **KLK3** | Kallikrein related peptidase 3, prostate-specific antigen, hydrolase, protease, serine protease, cancer-related genes, FDA approved drug targets. The gene is one of the fifteen kallikrein subfamily members located in a cluster on chromosome 19. It encodes a single-chain glycoprotein, a protease which is synthesized in the epithelial cells of the prostate gland and is present in seminal plasma. It is thought to function normally in the liquefaction of seminal coagulum, presumably by hydrolysis of the high molecular mass seminal vesicle protein. The serum level of this protein, called PSA in the clinical setting, is useful in the diagnosis and monitoring of prostatic carcinoma. |

### Additional data sources

Further sources of information may be used in the multi-omics assessment of a patient in diagnosing and predicting the risk posed by prostate cancer.

Imaging and histology data may further be incorporated into the assessment. One of the most common imaging modalities used in this way is MRI scanning, which can offer initial diagnosis of prostate cancer through providing an accurate visualisation of the volume and location of a tumour. Additionally, information on its potential growth characteristics can be estimated from visual characteristics elucidated through this imaging system. MRI is also relied upon for active surveillance of patients. MRI scans reinforce biological data and enables the clinicians to prepare for surgery.

Clinical data derived from questions asked by experienced clinicians, such as age, ethnicity, family history and the identification of particular symptoms, can also provide pointers which may be useful in predicting the course of a tumour found in the prostate. Therefore, this type of data is also useful in the final interpretation of the multi-omic assessment of prostate cancer.

### Machine Learning (ML) Approach

Multi-omics data obtained using the techniques discussed above are used in a machine learning approach to predict the current and/or future status of a cancer in a subject, as will now be described.

### Overview

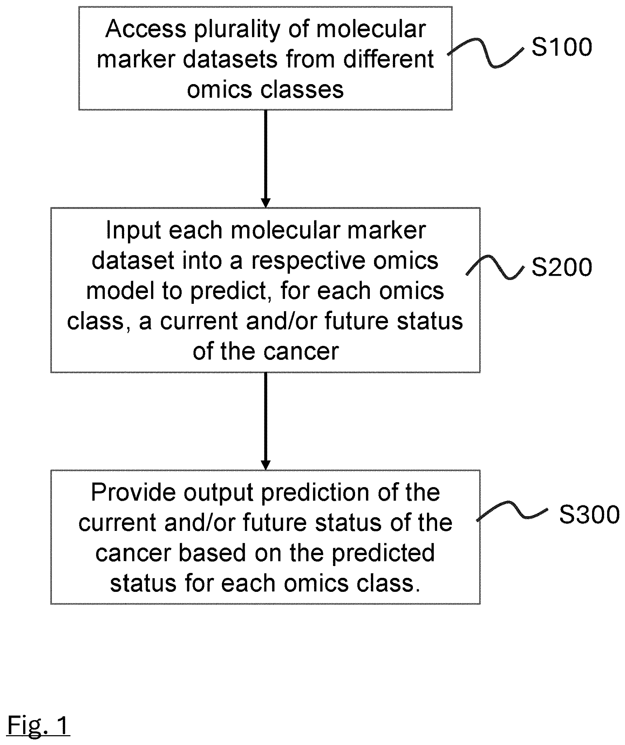

Figure 1 illustrates a flow diagram outlining the principal steps of a method of predicting the current and/or future status of a cancer in a subject according to an embodiment of the invention. At step S100, the method comprises accessing (e.g. from a database) a plurality of molecular marker datasets from different omics classes. Each molecular marker dataset represents the presence of one or more molecular markers from a respective omics class as determined within one or more biological samples of the subject. Typically, the plurality of molecular marker datasets comprises a genomics dataset, a transcriptomics dataset, an epigenomics dataset, and a proteomics dataset.

At step S200, each molecular marker dataset is input into a respective omics model to predict, for each omics class, a current and/or future status of the cancer in the subject. Each omics model is a trained machine learning or statistical model. The output prediction for each omics model is typically one or more risk scores indicative of the current and/or future status of the cancer within the subject. The omics models may receive additional input features, such as clinical characteristic data of the subject.

At step S300, the method provides an output prediction of the current and/or future status of the cancer based on the predicted status for each omics class. In some embodiments this may be a weighted combination of the individual predictions (e.g. risk scores) for each omics class that were obtained in step S200. The output prediction may take into account further data such as clinical characteristic data of the subject and/or imaging data.

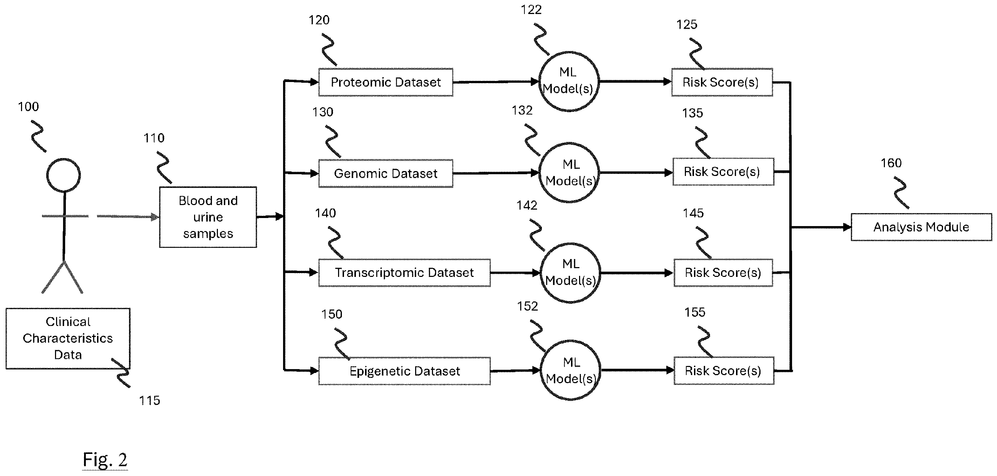

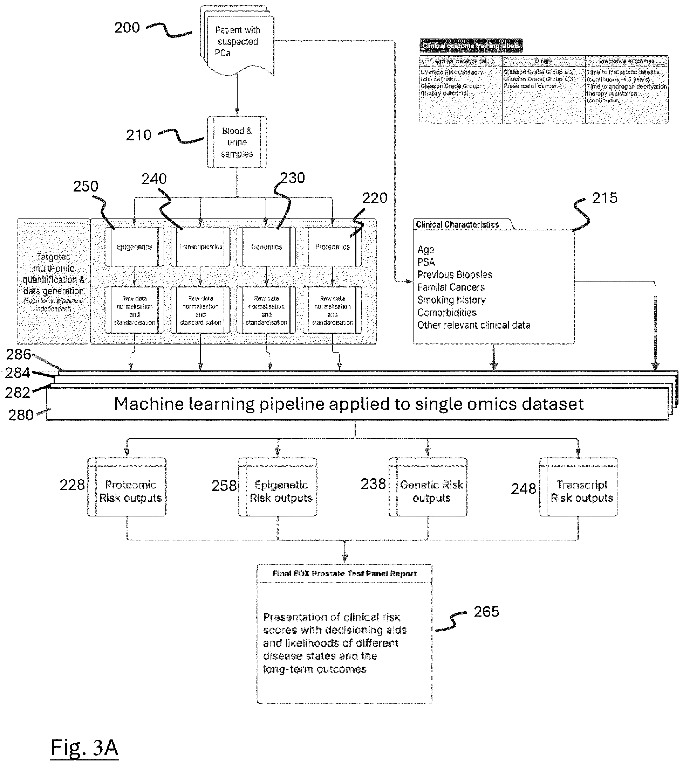

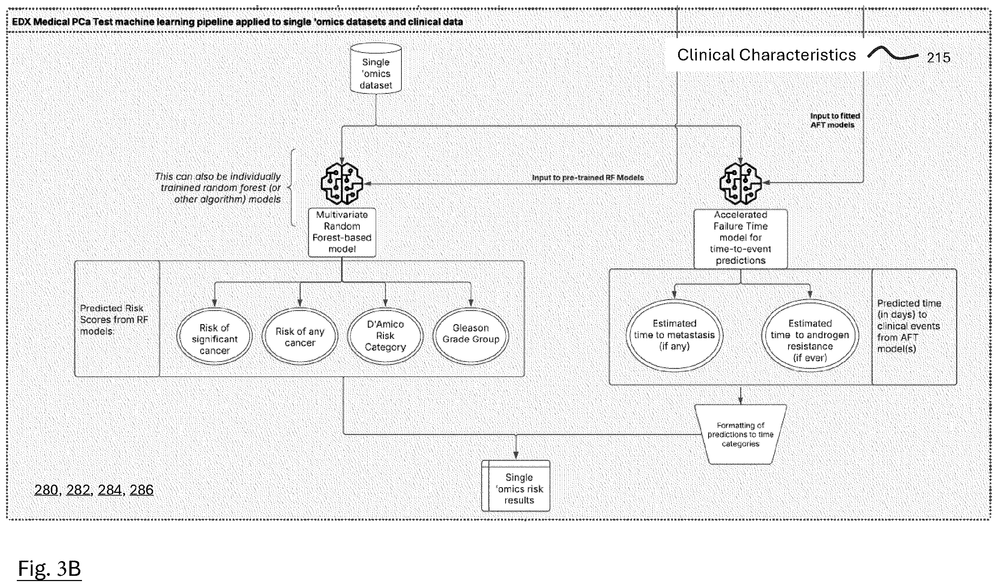

The steps of Figure 1 will now be described in more detail with reference to Figures 2, 3A and 3B.

Figure 2 illustrates a flow diagram schematically outlining a method for predicting prostate cancer according to an embodiment of the invention. Blood and urine samples 110 are collected from a patient 100 suspected of having prostate cancer. Clinical characteristics data 115 are also collected from the patient, including one or more of age, PSA levels, previous biopsies, family history and other comorbidities.

The blood and urine samples 110 are analysed *ex-vivo* to extract a plurality of distinct molecular marker datasets from different omics classes, using techniques known in the art and as has been described herein. Specifically, the biological samples 110 are analysed to obtain a proteomic dataset 120, a genomic dataset 130, a transcriptomic dataset 140, and an epigenetic dataset 150. The proteomic dataset 120 comprises protein markers quantitation; the genomic dataset 130 comprises mutations and genetic variations data; the transcriptomic dataset comprises gene expression data; and the epigenetics dataset comprise DNA methylation index data. The selected biomarkers used for each dataset obtained from the patient samples according to an embodiment of the invention are set out in Tables 1, 2, 3 and 4a herein.

In embodiments, the method may start by accessing (e.g. from a database) molecular marker datasets 120, 130, 140, 150 that have previously been obtained from one or more biological samples from a patient.

Each independent molecular marker dataset 120, 130, 140, 150 is then processed independently in its own machine learning pipeline to generate a prediction of the status of the prostate cancer within the patient 100, based on the respective molecular marker data. In other words, the proteomic dataset 120 is processed in its own ML pipeline to generate a prediction based on the proteomic data, and similarly for the genomics, transcriptomics and epigenetics datasets.

We take as an example the proteomic dataset 120. These data are input into one or more trained machine learning models 122 to generate one or more numerical risk scores 125 indicative of a prediction of the current or future status of the cancer within the patient 100. The raw data may be normalised and standardised for input into the ML model(s) as required. In some embodiments, information may be shared across the omics domains: for example a protein target may be used to normalise some or all of the methylation data. The machine learning model(s) 122 are trained to take as input the data from the proteomic dataset 120 and provide a numerical risk score indicative of one or more of the following clinical risk indicators or clinical end points:
- Risk of any cancer (e.g. as defined by any amount of positivity in biopsy data)
- Risk of clinically significant cancer (e.g. as defined by a diagnosis of greater than or equal to Gleason Grade Group 3)
- Gleason Grade Group
- D'Amico Risk Category (e.g. low, medium, high)
- Prostate Imaging-Reporting and Data System (PI-RADS) Score.

Preferably, the ML model(s) 122 are random forest model(s). In some embodiments a singular multivariate random forest model may be used to generate a risk score for each clinical risk indicator. However, in alternative embodiments, individually trained models for each clinical risk indictor may be used. In some embodiments, the ML model(s) may further take as input one or more of the clinical characteristics data 115 of the patient 100.

Thus, the output of the proteomic pipeline is one or more risk score(s) 125 indicative of the status of prostate cancer within the patient 100, based on the proteomic dataset 120. The genomics dataset 130, transcriptomics dataset 140 and epigenetics dataset 150 are each processed in their own independent pipelines in the corresponding manner to generate their own risk scores 135, 145, 155 for the same clinical risk indicators.

The risk scores 125, 135, 145 and 155 from each of the individual domains are then analysed in analysis module 160 to provide an output prediction of the patient's risk of prostate cancer based on the plurality of different omics classes (i.e. proteomics, genomics, transcriptomics, epigenomics). The analysis module 160 weights the risk scores from each of the omics classes to provide the output prediction. For example, the output prediction may be a weighted risk score for each of the clinical risk indicators predicted in the individual omics class pipelines. This weighting may be performed by inputting the outputs (e.g. risk scores) from each omics class pipeline into a regression model or using other analysis techniques. Additional data may be input into the analysis module to generate the integrated prediction, such as (additional) clinical characteristics data and/or MRI or ultrasound imaging data.

In some embodiments the analysis module 160 may generate a report for review by a medical practitioner or the patient to assess and/or determine treatment options, including one or more of:
- Risk scores from the different omics classes
- Predicted long-term cancer progression risks
- Decision making aids for treatment options.

Figures 3A and 3B illustrate the steps of a method for predicting prostate cancer according to a further embodiment of the invention. The methodology flow is similar to that illustrated in Figure 2, where the molecular datasets from each omics class are processed independently from each other to generate their own set of risk scores that are then used to generate a final output prediction.

As schematically illustrated in Figure 3A, blood and urine samples 210 are collected from a patient 200 suspected of having prostate cancer (e.g. having elevated PSA levels). The blood and urine samples are analysed *ex-vivo* to extract the proteomics, genomics, transcriptomics and epigenomics molecular marker datasets 220, 230, 240, 250. In embodiments, the method may start by accessing (e.g. from a database) molecular marker datasets 220, 230, 240, 250 that have previously been obtained from one or more biological samples from a patient.

Each distinct molecular marker dataset is then processed, along with clinical characteristics data 215, in an independent machine learning pipeline schematically illustrated at 280, 282, 284, 286. Thus, the proteomics dataset 220 is processed in its own discrete pipeline 280 to generate proteomic risk outputs 228. Similarly, the genomics, transcriptomics and epigenomics datasets are processed in their own respective independent pipelines 282, 284, 286 to generate genetic risk outputs 238, transcriptomics risk output 248 and epigenomic risk outputs 258. These risk outputs from the different omics domains are then used to provide an output prediction. In this embodiment the output prediction comprises a report 265.

Figure 3B illustrates the component parts of the machine learning pipelines 280, 282, 284, 286 in more detail. In this embodiment, each single omics pipeline is substantially the same (i.e. each omics dataset is processed using equivalent models), although it is envisaged that different pipelines may be used. As shown in Figure 3B, a single omics dataset is input into a multivariate random forest model together with the clinical characteristic data 215 to generate a plurality of risk scores for that omics domain. In alternative implementations, an individual random forest model for each clinical risk indicator may be used to provide a respective risk score for that risk indicator.

In the embodiment illustrated in Figure 3B, in addition to the predictive risk scores for the clinical risk indicators (risk of any cancer, risk of significant cancer, D'Amico risk category, Gleason Grade Group), each single omics molecular marker dataset is input into a prognostic model, in this case an Accelerated Failure Time (AFT) model. The AFT model may also take as input the clinical characteristics data from the patient. The AFT model predicts long term outcomes such as estimated time (e.g. in days or years) to metastasis (if cancer will spread and when), and estimated time (e.g. in days or years) to androgen resistance (if cancer will become resistant to hormonal treatment).

The predictions from the AFT model(s) may then be combined with the risk scores from the random forest model(s) to generate, for each omics class (in this case the proteomics class), a risk result.

In the embodiments described in Figures 2 and 3, the pipelines for each omics class have been described as outputting risk scores or results for the same clinical risk indicators. However, it is envisaged that in alternative embodiments, the outputs from each omics class may be indicative of different clinical risk indicators. For example, a dataset from one omics class may generate a risk score for "risk of cancer" only, whereas a dataset from a different omics class may generate a risk score for different or additional risk indicators (e.g. "risk of significant cancer", risk of any cancer", D'Amico Risk Category", and "Gleason Grade Group"). Similarly, the AFT models may only be applied on some of the datasets from different omics classes.

In the embodiments described above, the data obtained from the patient's blood and urine samples includes each of a proteomic dataset, a genomic dataset, a transcriptomic dataset and an epigenetic dataset. However, in general, two or more datasets from different omics classes may be used.

### Model Training and validation data

This section describes the training and validation data that will be used for each of the 'omics class models.

The training data comprises a dataset of well-characterised patients from previously quantified and characterised repositories. The training data may be obtained from publicly available datasets, such as the UK Biobank.

The training dataset(s) within each 'omics domain comprise a curated cohort of well-defined patients, with balanced proportions of biopsy outcomes and/or clinical risk categories as needed. The characterisation of these patients preferably includes one or more of:
- Extensive template-derived biopsy information, including detailed histopathological analysis quantifying the amount of disease present
- True multiparametric MRI (preferably comprising information from T1 and T2 weighting, diffusion-weighted imaging and dynamic contrast enhancement).
- PSA <50 ng/mL
- Eligible for treatment, with no co-occurring cancers.
- Not an extreme outlier in clinical risk category:
   ∘ For example a patient presenting with a PSA of 45 ng/mL but Gleason 3+3 on biopsy
- For predicting long term outcomes:
   ∘ Follow-up data is needed to determine metastatic disease status (rather than *de novo* metastatic disease)
   ∘ Treatment pattern data, including drug and therapy regimens

This dataset is not clinically representative by design. Advantageously, a cohort designed as above will have fewer patients, but with more information rather than more patients with less information and subsequently more variance that cannot be used for learning. Preferably two independent cohorts are used to develop/train the signatures to provide full external validation and a strongly validated model that will only require calibration

Internal cross-validation and/or resampling methods are used to develop robust models within each 'omics class, described below

In embodiments, each model is considered for external validation in clinically representative data. Validation over multiple, smaller cohorts from different clinical centres is more useful, and can incorporate variations to local practice to strengthen the evidence of predictive utility.

### Model Development

Input data within a given omics class comprises a matrix of numeric data, including quantitative count data (e.g. number of proteins in the case or the proteomics dataset, number of transcripts in the transcriptomics dataset etc.) and more qualitative index data (e.g. index or percentage of methylation against a known control or phosphorescence for the epigenomics dataset etc).

Predictive models are trained to predict the class likelihood of multiple clinically relevant outcomes in one training loop. These include:
- Presence of any prostate cancer, preferably as defined by any amount of positivity in biopsy data
- Presence of clinically relevant prostate cancer, preferably defined by a diagnosis of ≥ Gleason Grade Group 3
- Gleason Grade Group of disease, preferably including a negative for disease class
- D'Amico risk group category (a compound clinical risk grouping)
- PI-RADS score

Multivariate random forests are used for developing the predictive models, and so no scaling or normalisation of data are needed. Where categorical data are to be included (as predictor classes) they are preferably one-hot encoded as needed unless they can be considered ordinal and then treated as a bounded numerical value. For example, Low > Medium > High clinical risk (D'Amico) is seen as a continuous gradient and treated as a bounded continuous predictor between 0 - 3.

Although multivariate random forest models may be used, in alternative embodiments, individual models may be trained for each clinically relevant outcome (clinical risk indicator).

The outputs of models will reflect the classes to be predicted - a percentage likelihood between 0 and 100% of the outcome being trained, or the severity of disease present as described above.

The outputs of models will reflect the classes to be predicted - a percentage likelihood between 0 and 100% of the outcome being trained, or the severity of disease present as described above.

Bootstrap resampling is preferably used to determine the feature importance of each input biomarker, and measured alongside an artificially created "Shadow Feature" - a random feature created by permutation of another real biomarker (e.g. via the Boruta algorithm). This will enable poorly performing biomarkers to be removed for final model creation prior to external validation.

In some embodiments, additional (prognostic) models are trained in isolation, using the same input data to provide time-to-event predictions such as predicted time (e.g. in days or years) for one or mor of:
- Estimated time to metastasis (if any)
- Estimated time to androgen resistance (if ever).

These models may be Accelerated Failure Time models.

In some embodiments, additional models are trained in isolation, using the same input data to produce likelihoods for one or more of:
- The development of metastatic disease over long term follow-up
- The failure of specific therapy types or regimens.

### Computational System

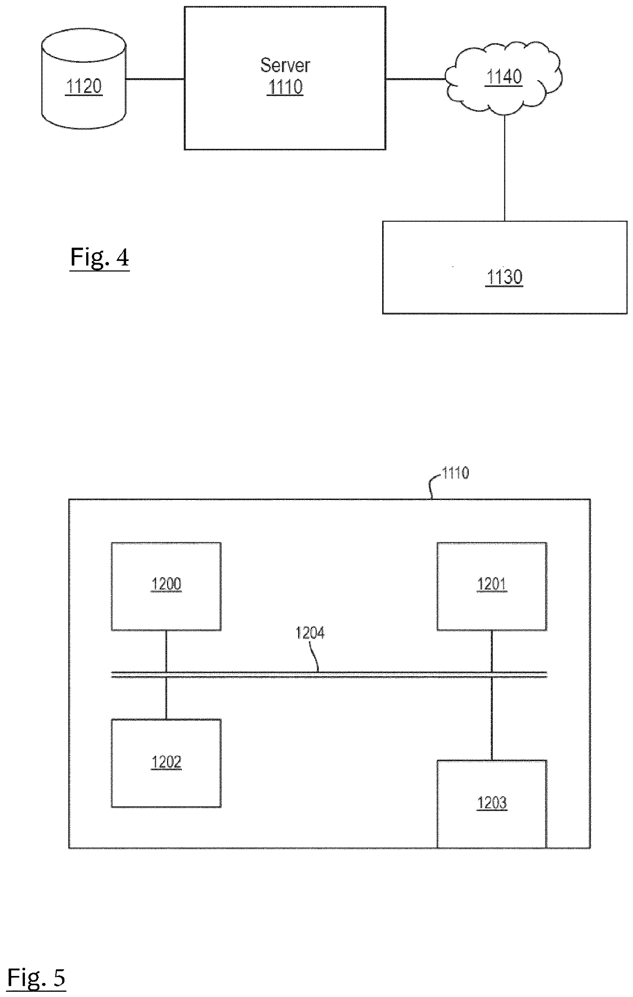

Figure 4 schematically illustrates an example of a system suitable for implementing embodiments of the invention. The system comprises at least one server 1110 which is in in communication with reference store 1120, such as a database. The server is also in communication with a client device 1130 over a communications network 1140 such the internet. The client device may be operated by a healthcare professional, such as a clinician, and may be a desktop computer or mobile device for example. The healthcare professional may send a request for a predicted status of a cancer in a patient to the server 1110 which is configured to implement embodiments of the invention as described herein to provide a prediction of the current and/or future status of the cancer. This prediction may then be communicated back to the client device 1130 for display to the healthcare professional, for example in the form of a report indicating risk scores and decisioning tools as described herein.

An example of a suitable server 1110 for implementing embodiments of the invention is shown in Figure 5. In this example, the server includes at least one microprocessor 1200, a memory 1201, an optional input/output device 1202, such as a keyboard and/or display, and an external interface 1203, interconnected via a bus 1204 as shown. In this example the external interface 1203 can be utilised for connecting the server 1110 to peripheral devices, such as the communications networks, reference data stores, other storage devices, or the like.

Although a single external interface 1203 is shown, this is for the purpose of example only, and in practice multiple interfaces using various methods (e.g. Ethernet, serial, USB, wireless or the like) may be provided.

In use, the microprocessor 1200 executes instructions in the form of applications software stored in the memory 1201 to allow the required processes to be performed, including providing a predicted current or future status of a cancer according to the methods described herein. The applications software may include one or more software modules, and may be executed in a suitable execution environment, such as an operating system environment, or the like.

Accordingly, it will be appreciated that the server 1100 may be formed from any suitable processing system, such as a suitably programmed client device, PC, web server, network server, or the like. In one particular example, the server 1100 is a standard processing system such as an Intel Architecture based processing system, which executes software applications stored on non- volatile (e.g., hard disk) storage, although this is not essential. However, it will also be understood that the processing system could be any electronic processing device such as a microprocessor, microchip processor, logic gate configuration, firmware optionally associated with implementing logic such as an FPGA (Field Programmable Gate Array), or any other electronic device, system or arrangement. Accordingly, whilst the term server is used, this is for the purpose of example only and is not intended to be limiting.

Whilst the server 1100 is a shown as a single entity, it will be appreciated that the server 1100 can be distributed over a number of geographically separate locations, for example by using processing systems and/or databases 1201 that are provided as part of a cloud based environment. Thus, the above described arrangement is not essential and other suitable configurations could be used. In a preferred embodiment, the processing systems are cloud based.

## Claims

1. A computer-implemented method of predicting the current and/or future status of a cancer in a subject suspected of having said cancer, comprising:
(a) accessing a plurality of molecular marker datasets, wherein each molecular marker dataset represents the presence of one or more molecular markers from a respective omics class as determined within one or more biological samples of the subject, wherein
the omics classes of the plurality of molecular marker datasets are different;
(b) inputting each molecular marker dataset into a respective omics model to predict, for each omics class, a current and/or future status of the cancer in the subject, wherein each omics model is a trained machine learning or statistical model; and
(c) providing an output prediction of the current and/or future status of the cancer in the subject, based on the predicted status of the cancer for each omics class obtained in step (b).

2. The method of claim 1, wherein the output of each omics model comprises a quantification, numerical score or categorisation indicative of the predicted current or future status of the cancer in the subject.

3. The method of claim 1 or claim 2, wherein the output of each omics model comprises a predicted risk score indicative of one or more clinical risk indicators, preferably where the one or more clinical risk indicators include one or more of:
(i) the presence of cancer in the subject;
(ii) the presence of clinically significant cancer in the subject;
(iii) a risk category, such as a D'Amico risk category, a Gleason Grade Group or a PI-RADS (Prostate Imaging-Reporting and Data System) score in the case of prostate cancer.

4. The method of any of the preceding claims, wherein the omics class of each of the molecular marker datasets is one of the following:
(i) genomics
(ii) transcriptomics
(iii) epigenomics
(iv) proteomics.

5. The method of any of the preceding claims, wherein the plurality of molecular marker datasets comprises a genomics dataset, a transcriptomics dataset, an epigenomics dataset, and a proteomics dataset.

6. The method of any of the preceding claims, wherein at least two of the molecular marker datasets are from the same biological sample.

7. The method of any of the preceding claims, wherein the step (c) of providing an output prediction of the status of the cancer in the subject comprises assigning a weighting to each predicted status to obtain the output prediction.

8. The method of claim 7 when dependent on claim 3, wherein the weighting is dependent on the clinical risk indicator.

9. The method of any of the preceding claims, wherein the step (c) of providing an output prediction of the status of the cancer in the subject comprises inputting the predicted status of the cancer for each omics class into an ensemble model, preferably wherein the ensemble model is a trained machine learning or statistical model.

10. The method of any of the preceding claims, wherein the input into respective omics model and/or the ensemble model further comprises one or more of:
(i) subject clinical characteristic data
(ii) imaging data, preferably MRI data or ultrasound data.

11. The method of claim 9 or claim 10, wherein the ensemble model is a regression model.

12. The method of any of the preceding claims, wherein each omics model is trained on a respective reference dataset comprising a plurality of reference subject profiles including:
(i) reference molecular marker data indicative of the presence of one or more molecular markers of the respective omics class from one or more biological samples obtained from the reference subject; and
(ii) one or more labels indicative of the present or future status of the cancer in the reference subject.

13. The method of claim 12, wherein the one or more labels indicative of the status of the cancer in the reference subject comprise one or more clinical risk indicators such as one or more of: a D'Amico risk category, a Gleason Grade Group, a presence of cancer, an imaging risk score.

14. The method of any of the preceding claims, wherein at least one omics model is a multivariate model.

15. The method of any of the preceding claims, wherein the cancer is prostate cancer.

16. The method of claim 15, wherein the plurality of molecular marker datasets comprises a genomics dataset comprising one or more of the molecular markers listed in Table 1.

17. The method of claim 15 or claim 16, wherein the plurality of molecular marker datasets comprises a transcriptomics dataset comprising one or more of the molecular markers listed in Table 3.

18. The method of any of claim 15 to 17, wherein the plurality of molecular marker datasets comprises an epigenomics dataset comprising one or more of the molecular markers listed in Table 2.

19. The method of any of claims 15 to 18, wherein the plurality of molecular marker datasets comprises a proteomics dataset comprising one or more of the molecular markers listed in Table 4a.

20. The method of any of the preceding claims, wherein each omics model comprises one of: a random forest model, a penalized regression model, a gradient boosted machine, a support vector machine or an artificial neural network.

21. The method of any of the preceding claims, wherein at least one of the plurality of molecular marker datasets is input into a respective prognostic model to generate one or more long term outcome predictions indicative of the future status of the cancer.

22. The method of any of the preceding claims, further comprising the initial steps of:
providing one or more biological samples from the subject; and
obtaining the plurality of molecular marker datasets from the one or more biological samples.

23. A computer-implemented method of predicting the current and/or future status of a cancer in a subject suspected of having said cancer, comprising:
(a) accessing a multi-omics dataset comprising molecular marker data representing the presence of one or more molecular markers from a plurality of different omics class as determined within the biological sample obtained from a subject; and
(b) inputting the multi-omics dataset into a trained machine learning or statistical model to predict a current and/or future status of the cancer in the subject based on the plurality of different omics classes.

24. A computer-implemented method of training a machine learning or statistical model to predict the current and/or future status of a cancer in a subject suspected of having said cancer, comprising:
(a) obtaining a reference dataset comprising a plurality of reference subject profiles including:
(i) reference molecular marker data representative of the presence of one or more molecular markers from an omics class as determined within one or more biological samples obtained from the reference subject; and
(ii) one or more labels indicative of the status of the cancer in the reference subject; and
(b) applying a machine learning or statistical model to the reference dataset to learn a relationship between the reference molecular marker dataset and the one or more labels.

25. The method of any of the preceding claims, wherein the subject is a human.

26. The method of any of the preceding claims, wherein the one or more biological samples include one of more of: a blood sample, a urine sample, a saliva sample, a tumor sample.

27. A system for predicting the current and/or future status of a cancer in a subject, comprising at least one processor in communication with at least one memory device, the at least one memory device having stored thereon instructions for causing the at least one processor to perform the method according to any of the preceding claims.

28. A computer program product comprising instructions which, when executed by a computer, cause the computer to perform the method of any of claims 1 to 26.
